(19) Europäisches Patentamt
European Patent Office
Office européen des brevets

(11) **EP 1 326 711 B1**

(12) # FASCICULE DE BREVET EUROPEEN

(45) Date de publication et mention de la délivrance du brevet:
**22.06.2005 Bulletin 2005/25**

(21) Numéro de dépôt: **01976375.4**

(22) Date de dépôt: **08.10.2001**

(51) Int Cl.[7]: **B01J 31/12**, C07C 269/00

(86) Numéro de dépôt international:
**PCT/FR2001/003093**

(87) Numéro de publication internationale:
**WO 2002/030565 (18.04.2002 Gazette 2002/16)**

(54) **UTILISATIONS DE DERIVES DE L'ETAIN COMME CATALYSEURS DE TRANSCARBAMATATION, COMPOSITIONS DE CARBAMATE COMPORTANT LEDIT CATALYSEUR ET PROCEDES DE TRANSCARBAMATATION**

VERWENDUNG VON ZINNDERIVATEN ALS TRANSCARBAMATIERUNGSKATALYSATOREN,CARABAMATEZUSAMMENSTELLUNGEN UND TRANSCARBAMATIERUNGSVERFAHREN

USE OF TIN DERIVATIVES AS CATALYSTS FOR TRANSFORMING CARBAMATES, CARBAMATE COMPOSITIONS COMPRISING SAID CATALYST AND METHOD FOR TRANSFORMING CARBAMATES

(84) Etats contractants désignés:
**AT BE CH CY DE DK ES FI FR GB GR IE IT LI LU MC NL PT SE TR**

(30) Priorité: **13.10.2000 FR 0013164**

(43) Date de publication de la demande:
**16.07.2003 Bulletin 2003/29**

(73) Titulaire: **RHODIA CHIMIE**
**92512 Boulogne Billancourt Cedex (FR)**

(72) Inventeurs:
- **BERNARD, Jean-Marie**
  **F-69440 MORNANT (FR)**
- **JOUSSEAUME, Bernard**
  **F-33400 TALENCE (FR)**
- **LAPORTE, Christian**
  **F-31320 CASTANET-TOLOSAN (FR)**
- **TOUPANCE, Thierry**
  **F-33400 TALENCE (FR)**

(74) Mandataire: **Ricalens, François**
**Rhodia Services,**
**Direction de la Propriété Industrielle,**
**40, rue de la Haie-Coq**
**93306 Aubervilliers Cedex (FR)**

(56) Documents cités:
**DE-A- 19 756 748**
**DE-C- 4 317 428**
**US-A- 6 010 976**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 1 326 711 B1

## Description

La présente invention a pour objet de nouveaux catalyseurs de transcarbamatation. Elle concerne plus spécifiquement l'utilisation de nouveaux catalyseurs à base d'étain.

En raison de leur forte réactivité et de leur relative toxicité, les isocyanates sont souvent utilisés sous une forme dérivée ou sous une forme masquée. Cette forme dérivée ou cette forme masquée présente l'inconvénient correspondant à ses avantages, c'est-à-dire qu'elle est peu réactive, aussi nécessite-t-elle l'utilisation de catalyseurs pour que la réaction puisse avoir lieu à des températures acceptables par l'industrie.

Les isocyanates masqués sont utilisables dans toutes les applications des isocyanates, à savoir les peintures, les vernis et plus généralement les revêtements, les colles et adhésifs, ainsi que certains polymères de spécialité.

Les catalyseurs les plus utilisés sont les alcanoates de dialcoylétain dont le plus connu est le dilaurate de dibutylétain. Cependant, pour certaines applications, le dilaurate de dibutylétain présente une activité insuffisante, aussi est-on obligé de l'utiliser à de très fortes concentrations.

Ces catalyseurs de transcarbamatation permettent de réaliser des polyuréthanes, notamment des polyuréthanes aliphatiques.

C'est pourquoi un des buts de la présente invention est de fournir un catalyseur de transcarbamatation qui soit plus actif que le dilaurate de dibutylétain.

Un autre but de la présente invention est de fournir un catalyseur du type précédent qui soit aussi utilisable pour la carbamatation des oximes.

Un autre but de la présente invention est de fournir un catalyseur de transcarbamatation qui soit utilisable pour transformer les carbamates de méthanol et les carbamates d'alcools primaires.

Ces buts, et d'autres qui apparaîtront par la suite, sont atteints au moyen de l'utilisation comme catalyseurs de transcarbamatation de composés de formule (I) générale suivante :

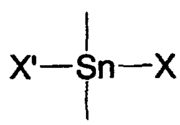

(I)

où :

- les liaisons « libres » de l'étain sont reliées à des groupes hydrocarbonés, avantageusement à des aryles ou à des alcoyles ;
- X' est choisi parmi les radicaux chlorure, bromure, iodure, thiocyanate, les radicaux sulfonates, avantageusement perfluorés sur le carbone porteur de la fonction sulfonate ;
- X est choisi parmi les valeurs de X' et parmi les radicaux de formule Y-Z ;
- Y est choisi dans le groupe des chalcogènes, avantageusement légers (c'est-à-dire oxygène et soufre) ;
- Z est choisi dans le groupe constitué par l'étain trisubstitué, le zinc monosubstitué, et les restes d'acides oxygénés après ignorance de la fonction OH.

Les composés de cette famille présentent une activité catalytique particulièrement intéressante. Parmi les composés de la présente famille, il convient de signaler plus particulièrement les composés où X' est un anion halogénure relativement lourd, c'est-à-dire chlorure, bromure ou iodure. Les deux premiers cités sont préférés, surtout le bromure. Les anions relativement volumineux dont l'acide associé présentent une acidité forte ou très forte donnent également de bons résultats surtout s'ils présentent une bonne liposolubilité. Parmi les acides donnant des anions engendrant de bonnes propriétés, on peut citer ceux qui présentent un $pK_a$ au plus égal à 2. Parmi ces acides, on peut citer les acides sulfoniques, les acides carboxyliques stables porteurs d'une fonction électro-attractrice sur le carbone carboxylique de manière que le $pK_a$ réponde à la contrainte ci-dessus. On peut notamment citer l'acide perfluoroacétique.

Un autre acide donnant des résultats particulièrement satisfaisants est l'acide thiocyanique ou plus exactement son anion, le thiocyanate. Les acides sulfoniques préférés sont ceux dont le carbone porteur de la fonction sulfonique, porte au moins deux atomes de fluor.

Parmi les membres les plus actifs de la famille définie par la formule (I), il convient de citer les composés symétriques, c'est-à-dire ceux où X répond à la formule Y-Z et où Z est le symétrique de X'-Sn-, en sorte que l'atome de chalcogène porte deux radicaux identiques. Dans la formule (1), les liaisons "libres" de l'étain sont avantageusement reliées à des groupes hydrocarbonés, c'est-à-dire comportant de l'hydrogène et du carbone (mais non nécessairement ne comportant que de l'hydrogène et du carbone). Ces groupes hydrocarbonés sont avantageusement choisis parmi

les aryles ou les alcoyles (pris dans leur sens étymologique d'un alcool dont on ignore ladite fonction alcool) ; les alcoyles sont préférés.

L'ensemble des radicaux doit être tel que le nombre de carbone des composés de formule (I) ne comporte au plus 50 atomes de carbone, de préférence 25 atomes de carbone.

La liaison "libre" du zinc est en général reliée à un composé hydrocarboné, avantageusement à l'anion d'un acide oxygéné, avantageusement carboxylique.

Ces catalyseurs sont avantageusement utilisés en quantité au moins égale à 0,5‰ et au plus égale à 5% en équivalent de fonction carbamate, de préférence entre 1‰ et 2%.

La réaction de transcarbamatation dépend des alcools et des carbamates utilisés. Elle est toutefois comprise entre 100 et 200°C, de préférence entre 120 et 180°C. Les carbamates utilisés sont ceux issus de la réaction d'une fonction isocyanate avec une fonction hydroxyle. Parmi les fonctions hydroxyles, il convient de citer les fonctions alcools surtout celles des alcools volatils à la température de réaction (température d'ébullition sous pression atmosphérique) et plus particulièrement le méthanol.

Parmi les autres fonctions hydroxyles que l'on peut citer comme présentant un avantage particulier, les fonctions phénols, les fonctions hydroxyles greffées sur un atome d'azote comme les hydroxy-imides, les oximes.

Les alcools qui se substituent à ces dérivés hydroxylés sont avantageusement des polyols (surtout di- et/ou triols), avantageusement primaires.

Les masses moléculaires peuvent varier selon un large domaine selon la forme du revêtement utilisé. Les masses moléculaires sont relativement élevées pouvant aller environ jusqu'à 20.000 lorsque l'on met en oeuvre les catalyseurs selon la présente invention dans une peinture poudre, en revanche pour les applications les plus classiques, les polyols dépassent rarement une masse moléculaire de 3.000 environ.

Les masses moléculaires auxquelles il est fait référence sont des masses moléculaires moyennes en nombre $M_n$ et sont définies par la technique de perméation de gel connue de "l'homme de métier". Plus spécifiquement, la masse moléculaire est déterminée par chromatographie par perméation de gel (GPC). La technique utilise comme gels, deux gels de polystyrène (ultrastyragel® à $10^4$ et 500 Ä), le THF comme solvant et le soufre comme standards.

Les isocyanates donnant naissance ou correspondant aux carbamates préférés sont au moins partiellement des isocyanates aliphatiques, c'est-à-dire que la fonction isocyanate considérée est reliée par l'azote au squelette de la molécule isocyanate par un atome de carbone d'hybridation $sp^3$.

Il est en outre souhaitable que, dans la structure ou des isocyanates monomères (c'est-à-dire des isocyanates qui sont oligomérisés par la suite et dont les plus courants sont l'hexaméthylènediisocyanate et le composé désigné par le terme isophoronediisocyanate ou IPDI), la partie du squelette reliant deux fonctions isocyanates comporte au moins un enchaînement polyméthylène $(CH_2)_\pi$ où $\pi$ représente un entier de 2 à 10, avantageusement de 4 à 8. Cette préférence joue sur les performances mécaniques. Quand il y a plusieurs enchaînements, ces derniers peuvent être semblables ou différents. En outre, il est souhaitable que, dans un monomère, ces enchaînements polyméthylène soient libres en rotation et donc exocycliques. Lorsque l'on utilise des prépolymères, ou oligomères, issus de plus d'un monomère, il est souhaitable que la condition relative à cet enchaînement polyméthylène se retrouve dans l'un au moins de ces monomères.

Les polyisocyanates préférés sont ceux qui présentent au moins une fonction isocyanate aliphatique. En d'autres termes, au moins une fonction isocyanate masquée selon l'invention est reliée au squelette par l'intermédiaire d'un carbone de type $sp^3$ portant avantageusement un atome d'hydrogène, de préférence deux. Il est souhaitable que ledit carbone de type $sp^3$ soit lui-même porté par un carbone de type $sp^3$ et avantageusement muni d'un, de préférence de deux atomes d'hydrogène, et ce pour éviter que la fonction isocyanate considérée soit en position néopentylique. En d'autres termes, il est conseillé de choisir comme monomère (lesquels sont, en général, porteurs de deux fonctions isocyanates) au moins un composé qui porte au moins une fonction aliphatique qui ne soit ni secondaire ou tertiaire, ni néopentylique.

En cas de mélange obtenu à partir de plusieurs (en général deux) types de monomères, il est préférable que celui ou ceux des monomères qui répondent aux conditions ci-dessus et/ou (avantageusement "et") à la condition sur la présence d'enchaînement polyméthylène $(CH_2)_\pi$ représentent au moins 1/3, avantageusement 1/2, de préférence 2/3 des fonctions isocyanates masquées. Ainsi au cours de l'étude selon la présente invention, il a été obtenu d'excellents résultats avec des mélanges comportant 2/3 d'HMDT ("trimère" d'Hexaméthylène diisocyanate) avec de l'IPDI ou de l'IPDT ("trimère" d'IPDI) les deux étant masqués selon l'invention (le nBDI, norbornane diisocyanate, et son trimère sont similaires).

On préfère bien sûr le cas où la totalité des isocyanates sont aliphatiques, et même répondent au critère ci-dessus.

Selon la présente invention, l'isocyanate masqué, pur ou en mélange, est issu d'un polyisocyanate, c'est-à-dire possédant au moins deux fonctions isocyanates, avantageusement plus de deux (possibilités de valeurs fractionnaires puisqu'il s'agit en général de mélange d'oligomères plus ou moins condensés), lequel est lui-même le plus souvent issu d'une précondensation ou d'une prépolymérisation de diisocyanate unitaire (parfois qualifié dans la présente description de "monomère").

La masse moléculaire moyenne de ces prépolymères ou de ces précondensats est en général au plus égale à 2.000 (un chiffre significatif), plus couramment à 1.000 (un chiffre significatif, de préférence deux).

Ainsi, parmi les polyisocyanates utilisés pour l'invention, on peut citer ceux du type biuret et ceux dont la réaction de di- ou trimérisation a conduit à des cycles à quatre, cinq ou six chaînons. Parmi les cycles à six, on peut citer les cycles isocyanuriques issus d'une homo- ou d'une hétérotrimérisation de divers diisocyanates seuls, avec d'autre isocyanate(s) [mono-, di-, ou polyisocyanate(s)] ou avec du gaz carbonique (ou bioxyde de carbone), dans ce cas on remplace un azote du cycle isocyanurique par un oxygène. Les oligomères à cycles isocyanuriques sont préférés.

Parmi les monomères les plus intéressants, il convient de citer, d'une part ceux qui présentent un enchaînement polyméthylène tel que défini plus haut exocyclique, évidemment y compris non cyclique, parmi lesquels on peut citer le tétraméthylènediisocyanate éventuellement substitué par un groupe alcoyle avantageusement d'au plus quatre atomes de carbone, de préférence d'au plus deux atomes de carbone ; le pentaméthylènediisocyanate éventuellement substitué par un groupe alcoyle, avantageusement d'au plus quatre atomes de carbone, de préférence d'au plus deux atomes de carbone et l'hexaméthytènediisocyanate. Comme monomères de nature cycloaliphatique qui sont de préférence utilisés en association avec des isocyanates ayant des enchaînements polyméthylènes exocycliques, ou non cycliques, on peut citer les monomères et les composés issus des monomères suivants :

→ Les composés correspondant à l'hydrogénation du ou des noyaux aromatiques porteurs des fonctions isocyanates de monomères d'isocyanates aromatiques, et notamment du TDI (toluènediisocyanate) et des diisocyanates diphényles, le composé connu sous le sigle $H_{12}$MDI et les divers BIC [bis(isocyanatométhylcyclohexane)] ; et surtout
→ le norbomanediisocyanate souvent appelé par son sigle NBDI ;
→ l'isophoronediisocyanate ou IPDI ou 3-isocyanatométhyl-3,5-triméthylcyclohexylisocyanate.

La présente invention a également pour objet des compositions d'isocyanates masqués sous la forme de carbamates (lato sensu, c'est-à-dire les fonctions correspondant à la séquence -N(R)- CO-O- où R est un radical hydrocarboné, en général alcoyle, voire aryle, ou plus fréquemment un hydrogène) et comportant en outre un catalyseur de formule générale (I) suivante :

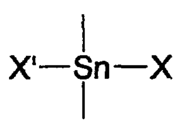 (I)

où :

les liaisons « libres » de l'étain sont reliées à des groupes hydrocarbonés, avantageusement à des aryles ou à des alcoyles ;
X' est choisi parmi les radicaux chlorure, bromure, iodure, thiocyanate, les radicaux sulfonates, avantageusement perfluorés sur le carbone porteur de la fonction sulfonate ;
X est choisi parmi les valeurs de X' et parmi les radicaux de formule Y-Z ;
Y est choisi dans le groupe des chalcogènes, avantageusement légers (c'est-à-dire oxygène et soufre) ;
Z est choisi dans le groupe constitué par l'étain trisubstitué, le zinc monosubstitué, et les restes d'acides oxygénés après ignorance de la fonction OH.

Ces isocyanates masqués sous la forme de carbamates seront désignés dans la suite de la description sous le terme de carbamates.

Ces compositions, qui sont utilisables dans l'industrie de la peinture et plus généralement des revêtements, y compris les adhésifs, comportent en général, en outre des alcools ou des apolyalcools, ou polyols qui sont décrits ci-dessus.

Ces fonctions alcools sont avantageusement primaires.

En effet, les alcools primaires réagissent, en général, plus rapidement que les alcools secondaires. Les carbamates décrits ci-dessus sont avantageusement des carbamates de composés hydroxylés choisis parmi les alcools volatils, à une température au moins égale à 150°C et parmi les agents de masquage hydroxylés. Les agents de masquage hydroxylés, lorsqu'il ne s'agit pas d'alcool, même lato sensu, c'est-à-dire les alcools vinyliques ou les phénols, sont en général des composés comportant la liaison -N-O-H. En d'autres termes, il s'agit essentiellement de composés où la fonction hydroxyle est portée par l'azote. Parmi les familles de ces composés, on peut citer les hydroxyimides et les oximes notamment les oximes visés par la demande de brevet européen (confère la demande au nom de la demanderesse publiée sous le numéro EP-A-0 869 982).

Les oximes et les hydroxyimides constituent une famille d'agents masquants couramment utilisés.

Une autre famille d'agents masquants présentant un certain nombre d'intérêts est constituée par les phénols, avantageusement présentant une fonction électro-attractrice sur le noyau (voir en particulier la demande de brevet européenne au nom de la demanderesse publiée sous le N° EP-A-0 680 984 et la demande internationale publiée sous le N° EP-A-0 998 510).

Lorsqu'elles sont utilisées comme liant pour peinture, les compositions comportent les additifs usuels pour cet usage.

La présente invention vise également un procédé de transcarbamatation où l'on utilise comme catalyseur de transcarbamatation les composés de formule (I) :

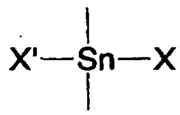

(I)

où :

les liaisons « libres » de l'étain sont reliées à des groupes hydrocarbonés, avantageusement à des aryles ou à des alcoyles ;
X' est choisi parmi les radicaux chlorure, bromure, iodure, thiocyanate, les radicaux sulfonates, avantageusement perfluorés sur le carbone porteur de la fonction sulfonate ;
X est choisi parmi les valeurs de X' et parmi les radicaux de formule Y-Z ;
Y est choisi dans le groupe des chalcogènes, avantageusement légers (c'est-à-dire oxygène et soufre) ;
Z est choisi dans le groupe constitué par l'étain trisubstitué, le zinc monosubstitué, et les restes d'acides oxygénés après ignorance de la fonction OH.

Avantageusement, l'un des X' ou X est choisi parmi les sulfonates perfluorés sur le carbone porteur de la fonction sulfonate. Les radicaux de ces sulfonates répondent avantageusement à la formule ci-après :

$$GEA\text{-}(CX_2)_p\text{-}$$

où :

les X, semblables ou différents, représentent un chlore, un fluor ou un radical de formule $C_nF_{2n+1}$ avec n entier au plus égal à 5 de préférence à 2, avec la condition qu'au moins un des X soit fluor, fluor avantageusement porté par le carbone relié au soufre ;
p représente un entier au plus égal à 2 ;
GEA représente un groupe électro-attracteur (c'est-à-dire sigma p supérieur à zéro, avantageusement à 0,1, de préférence à 0,2) dont les éventuelles fonctions sont inertes dans les conditions de la réaction, avantageusement fluor ou un reste perfluoré de formule $C_nF_{2n+1}$, avec n entier au plus égal à 8, avantageusement à 5, le nombre total de carbone de Rf étant avantageusement compris entre 1 et 15, de préférence entre 1 et 10.

Avantageusement, X et X' sont choisis parmi les composés de formule ci-dessus.

Ces catalyseurs permettent de réaliser la réaction à des températures inférieures à 200°C, avantageusement inférieures à 180°C.

En général pour obtenir une cinétique suffisante, il convient de se placer à une température au moins égale à 100°C, de préférence à une température au moins égalé à 120°C.

Les exemples non limitatifs suivants illustrent l'invention. Ces exemples, afin d'éviter tous problèmes d'interaction, ont sensiblement porté sur des isocyanates monofonctionnels. On a utilisé l'isocyanate de n-hexyle.

<u>Exemple</u>

Mode opératoire général :

Choix du modèle réactionnel

Le choix s'est porté sur l'utilisation d'isocyanates aliphatiques qui sont plus coûteux mais qui donnent accès à des polyuréthanes présentant :

- une meilleures résistance à l'U.V. ;
- une couleur plus stable ;
- une excellente tenue en usage externe.

L'action d'alcools primaires donne des carbamates très stables thermiquement par rapport aux isocyanates aromatiques.

Le méthanol a été choisi en tant qu'agent bloquant de l'isocyanate d'alcoyle. En effet, cet alcool présente un coût peu élevé, une masse moléculaire faible ainsi qu'une toxicité limitée.

Un tel agent bloquant favorise une limitation des C.O.V. par rapport aux autres agents. Cette méthodologie s'inscrit bien dans le cadre du développement d'une chimie plus propre, axe privilégié de la recherche contemporaine.

Réactifs mis en jeu

L'isocyanate d'alcoyle choisi est l'isocyanate d'hexyle. Son blocage est réalisé très simplement par le méthanol sous chauffage classique pendant 4 heures.

La réaction conduit à la formation du N-hexylméthyluréthane qui servira de réactif de départ au cours de la réaction de transcarbamatation.

$$n\text{-hex-N=C=O} + \text{MeOH} \rightarrow n\text{-hex-NH-CO-OMe}$$

L'alcool primaire choisi pour réaliser la transcarbamatation est l'octan-1-ol. Son action sur l'isocyanate d'hexyle sous chauffage classique pendant 4 heures conduit à la formation du produit d'arrivée : le N-hexyloctyluréthane.

$$n\text{-hex-N=C=O} + \text{n-OctOH} \rightarrow n\text{-hex-NH-CO-O-}n\text{-Oct}$$

La caractérisation de ce produit par les techniques d'analyse classique a été nécessaire pour réaliser un suivi cinétique de la réaction de transcarbamatation par chromatographie en phase gazeuse (temps de rétention et coefficient de réponse).

Etude cinétique

La réaction de transcarbamatation étudiée conduite en l'absence de solvant et en présence de 1% molaire de catalyseur (pour avoir une réaction rapide) est schématisée ci-dessous ;

$$\underset{\textbf{carba 1}}{n\text{-hex-NH-CO-OMe}} + \text{OctOH} \xrightarrow{\text{Cat.}} \underset{\textbf{carba 2}}{n\text{-hex-NH-CO-Oct}} + \text{MeOH}$$

- Carba 1 : 3,14 mmoles (500 mg)
- OctOH : 1 éq. ou 10 éq.
- Référence interne (hexadécane) : 0,5 éq.
- Cat. : 1% mol.
- 130°C

Résultats cinétiques de différents catalyseurs testés

| Catalyseur | k en $mol^{-1}.L.\ min^{-1}$ | Rdt (%) |
|---|---|---|
| $Bu_2Sn(laurate)_2$ Référence | $1,10.10^{-3}$ | 26 |
| $Bu_2Sn(2\text{-éthylhexanoate})_2$ Comparatif | $7,32.10^{-4}$ | 27 |
| BuSn(2-éthylhexanoate)3 Comparatif | $3,34.10^{-4}$ | 15 |
| $Bu_2SnCl_2$ | $9,73.10^{-4}$ | 34 |
| $Bu_2Sn(OMe)_2$ Comparatif | $5,18.10^{-4}$ | 17 |
| **$Bu_2SnCl(OAc)$** | **$2,30.10^{-3}$** | **53** |
| **$Bu_2SnBr(OAc)$** | **$2,57.10^{-3}$** | **55** |
| **$Bu_2Sn(NCS)_2$** | **$3,10.10^{-3}$** | **63** |

En ce qui concerne les catalyseurs témoins tels que les diacétate et dilaurate de dibutylétain, ces derniers affichent une activité catalytique très moyenne avec des rendements en fin de réaction respectivement de 43 et 26%.

Ensuite, on observe une série de composés à ligand alcanoate, halogène et méthoxy qui présentent une efficacité relativement peu importante.

Les composés mixtes halogène/acétate font preuve d'une excellente activité catalytique avec de bons rendements en fin de réaction. Cependant ces composés sont, en fait, des mélanges entre le composé d'étain IV et des distannoxanes symétrique et mixte correspondants. Ci-après, nous verrons que ces derniers sont très performants.

Enfin, le diisothiocyanate de dibutylétain s'avère le composé le plus actif de ce tableau avec une constante de vitesse de $3,10.10^{-3}$ $mol^{-1}.L.min^{-1}$ et un rendement final de 63 %.

Catalyseurs à base de distannoxanes

| Catalyseur | K en $mol^{-1}.L.\ min^{-1}$ | Rdt (%) |
|---|---|---|
| $[(AcO)Bu_2Sn]_2O$ | $1,39.10^{-3}$ | 42 |
| $[ClBu_2Sn]_2O$ | $1,84.10^{-3}$ | 51 |
| $[(NCS)Bu_2Sn]_2O$ | $3,54.10^{-3}$ | 62 |
| **$[BrBu_2Sn]_2O$** | **$5,10.10^{-3}$** | **68** |
| **$[BrBu_2Sn]_2O$ (0,5 mol)** | **$2,93.10^{-3}$** | **65** |
| **$[BrBu_2Sn]_2O$ (Qités*10)** | **$2,48.10^{-3}$** | **59** |
| *$(AcO)Bu_2Sn\text{-}O\text{-}Zn\text{-}OC_5H_{11}$* | $1,12.10^{-3}$ | 39 |

Dans le cadre de la réaction de transcarbamatation, ce type de composés se montre aussi d'une efficacité remarquable. C'est le 1,3-dibromotétrabutyldistannoxane qui s'avère le plus efficace ici, avec une constante de vitesse de $5,10.10^{-3}$ $mol^{-1}.L.min^{-1}$ et un rendement final de 68%.

Notons que même utilisé à 0,5% molaire ou avec des quantités de réactifs 10 fois supérieures, les résultats sont toujours aussi bons.

Néanmoins, il apparaît que ce type de composé conduit à la formation d'isocyanate en quantité non négligeable dans le milieu réactionnel.

## Revendications

1. Utilisation comme catalyseur de transcarbamatation de composés répondant à la formule (I) générale suivante :

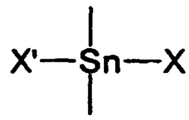 (I)

   où :

   - les liaisons « libres » de l'étain sont reliées à des groupes hydrocarbonés, avantageusement à des aryles ou à des alcoyles ;
   - X' est choisi parmi les radicaux chlorure, bromure, iodure, thiocyanate, les radicaux sulfonates, avantageusement perfluorés sur le carbone porteur de la fonction sulfonate ;
   - X est choisi parmi les valeurs de X' et parmi les radicaux de formule Y-Z ;
   - Y est choisi dans le groupe des chalcogènes, avantageusement légers c'est-à-dire oxygène et soufre ;
   - Z est choisi dans le groupe constitué par l'étain trisubstitué, le zinc monosubstitué, et les restes d'acides oxygénés après ignorance de la fonction OH.

2. Utilisation selon la revendication 1, **caractérisée par le fait que** X a la même valeur que X'.

3. Utilisation selon les revendications 1 et 2, **caractérisée par le fait que** l'un au moins des X ou X' est choisi parmi les halogénures et les thiocyanates.

4. Utilisation selon la revendication 1, **caractérisée par le fait que** Z a la même valeur que :

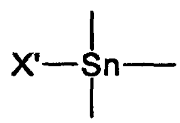

5. Composition **caractérisée par le fait qu'**elle comporte au moins un carbamate susceptible d'être obtenu par l'action d'un composé hydroxylé sur un isocyanate avantageusement aliphatique, et au moins un composé de formule générale (I) suivante :

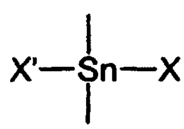 (I)

   où :

   - les liaisons « libres » de l'étain sont reliées à des groupes hydrocarbonés, avantageusement à des aryles ou à des alcoyles ;
   - X' est choisi parmi les radicaux chlorure, bromure, iodure, thiocyanate, les radicaux sulfonates, avantageusement perfluorés sur le carbone porteur de la fonction sutfonate ;
   - X est choisi parmi les valeurs de X' et parmi les radicaux de formule Y-Z ;
   - Y est choisi dans le groupe des chalcogènes, avantageusement légers c'est-à-dire oxygène et soufre ;
   - Z est choisi dans le groupe constitué par l'étain trisubstitué, le zinc monosubstitué, et les restes d'acides oxygénés après ignorance de la fonction OH.

6. Composition selon la revendication 5, **caractérisée par le fait qu'**elle comporte en outre un alcool ou un polyalcool avantageusement primaire.

7. Composition selon les revendications 5 et 6, **caractérisée par le fait que** ledit composé hydroxylé est choisi parmi les alcools volatils à une température au moins égale à 150°C et parmi les agents de masquage hydroxylés.

8. Composition selon les revendications 5 à 7, **caractérisée par le fait que** ledit composé hydroxylé est le méthanol.

9. Composition selon les revendications 5 à 7, **caractérisée par le fait que** ledit composé hydroxylé est choisi parmi les agents masquants, avantageusement parmi les phénols et les oximes.

10. Procédé de transcarbamatation, **caractérisé par le fait que** l'on utilise comme catalyseur de transcarbamatation les composés de formule (I) :

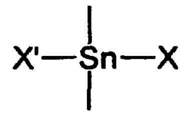

(I)

où:

- les liaisons « libres » de l'étain sont reliées à des groupes hydrocarbonés, avantageusement à des aryles ou à des alcoyles ;
- X' est choisi parmi les radicaux chlorure, bromure, iodure, thiocyanate, les radicaux sulfonates, avantageusement perfluorés sur le carbone porteur de la fonction sulfonate ;
- X est choisi parmi les valeurs de X' et parmi les radicaux de formule Y-Z ;
- Y est choisi dans le groupe des chalcogènes, avantageusement légers c'est-à-dire oxygène et soufre ;
- Z est choisi dans le groupe constitué par l'étain trisubstitué, le zinc monosubstitué, et les restes d'acides oxygénés après ignorance de la fonction OH.

11. Procédé selon la revendication 10, **caractérisé par le fait que** la réaction de transcarbamatation est menée à une température comprise entre 100 et 200°C, avantageusement entre 120 et 180°C.

**Patentansprüche**

1. Verwendung von Verbindungen, die der folgenden allgemeinen Formel (I) entsprechen:

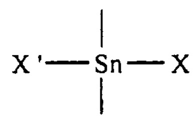

(I),

in der:

- die «freien » Bindungen des Zinns an Kohlenwasserstoffgruppen und vorteilhafterweise an Aryl- oder Alkylgruppen gebunden sind;
- X' ausgewählt ist unter den Chlorid-, Bromid-, Iodid- und Thiocyanatresten sowie den Sulfonatresten, deren Kohlenstoffatom, das die Sulfonatfunktion trägt, vorteilhafterweise perfluoriert ist,
und
- X unter den als Bedeutungen von X' genannten Resten sowie den Resten der Formel Y-Z ausgewählt ist, wobei:
  - Y unter den Chalcogenatomen und vorteilhafterweise den leichten Chalcogenatomen, das heißt dem Sauerstoff und dem Schwefel, ausgewählt ist
  und
  - Z unter trisubstituiertem Zinn, monosubstituiertem Zink und den Säureresten, die unter Ausnahme der OH-Funktion oxidiert sind, ausgewählt ist,

als Transcarbamoylierungskatalysatoren.

**2.** Verwendung nach Anspruch 1, **dadurch gekennzeichnet, dass** X dieselbe Bedeutung hat wie X'.

**3.** Verwendung nach Anspruch 1 oder 2, **dadurch gekennzeichnet, dass** zumindest einer der beiden Reste X und X' unter den Halogeniden und den Thiocyanaten ausgewählt ist.

**4.** Verwendung nach Anspruch 1, **dadurch gekennzeichnet, dass** Z dieselbe Bedeutung hat wie:

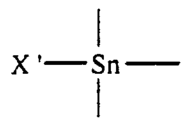

**5.** Zusammensetzung, **dadurch gekennzeichnet, dass** sie mindestens ein Carbamat, welches durch Umsetzung eines vorteilhafterweise aliphatischen Isocyanats mit einer hydroxylierten Verbindung erhalten werden kann, und mindestens eine Verbindung der folgenden allgemeinen Formel (I) enthält:

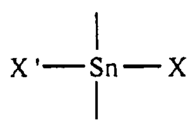

(I),

in der:

- die «freien» Bindungen des Zinns an Kohlenwasserstoffgruppen und vorteilhafterweise an Aryl- oder Alkylgruppen gebunden sind;
- X' ausgewählt ist unter den Chlorid-, Bromid-, Iodid- und Thiocyanatresten sowie den Sulfonatresten, deren Kohlenstoffatom, das die Sulfonatfunktion trägt, vorteilhafterweise perfluoriert ist,
  und
- X unter den als Bedeutungen von X' genannten Resten sowie den Resten der Formel Y-Z ausgewählt ist, wobei:

  - Y unter den Chalcogenatomen und vorteilhafterweise den leichten Chalcogenatomen, das heißt dem Sauerstoff und dem Schwefel, ausgewählt ist
    und
  - Z unter trisubstituiertem Zinn, monosubstituiertem Zink und den Säureresten, die unter Ausnahme der OH-Funktion oxidiert sind, ausgewählt ist.

**6.** Zusammensetzung nach Anspruch 5, **dadurch gekennzeichnet, dass** sie des Weiteren einen vorteilhafterweise primären Alkohol oder Polyalkohol enthält.

**7.** Zusammensetzung nach Anspruch 5 oder 6, **dadurch gekennzeichnet, dass** die besagte hydroxylierte Verbindung unter den Alkoholen, die bei einer Temperatur von mindestens 150 °C flüchtig sind, und den hydroxylierten Maskierungsmitteln ausgewählt ist.

**8.** Zusammensetzung nach einem der Ansprüche 5 bis 7, **dadurch gekennzeichnet, dass** es sich bei der besagten hydroxylierten Verbindung um Methanol handelt.

**9.** Zusammensetzung nach einem der Ansprüche 5 bis 7, **dadurch gekennzeichnet, dass** die besagte hydroxylierte Verbindung unter den Maskierungsmitteln und vorteilhafterweise unter den Phenolen und den Oximen ausgewählt ist.

**10.** Transcarbamoylierungsverfahren, **dadurch gekennzeichnet, dass** als Transcarbamoylierungskatalysatoren die

Verbindungen der Formel (I):

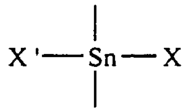 (I),

in der:

- die « freien » Bindungen des Zinns an Kohlenwasserstoffgruppen und vorteilhafterweise an Aryl- oder Alkylgruppen gebunden sind;
- X' ausgewählt ist unter den Chlorid-, Bromid-, Iodid- und Thiocyanatresten sowie den Sulfonatresten, deren Kohlenstoffatom, das die Sulfonatfunktion trägt, vorteilhafterweise perfluoriert ist,
  und
- X unter den als Bedeutungen von X' genannten Resten sowie den Resten der Formel Y-Z ausgewählt ist, wobei:

  - Y unter den Chalcogenatomen und vorteilhafterweise den leichten Chalcogenatomen, das heißt dem Sauerstoff und dem Schwefel, ausgewählt ist
    und
  - Z unter trisubstituiertem Zinn, monosubstituiertem Zink und den Säureresten, die unter Ausnahme der OH-Funktion oxidiert sind, ausgewählt ist,

verwendet werden.

**11.** Verfahren nach Anspruch 10, **dadurch gekennzeichnet, dass** die Transcarbamoylierungsreaktion bei einer Temperatur von 100 bis 200 °C und vorteilhafterweise von 120 bis 180 °C durchgeführt wird.

## Claims

**1.** Use as transcarbamation catalyst of compounds corresponding to the following general formula (I):

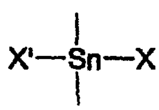 (I)

where:

- The "free" bonds of the tin are connected to hydrocarbonaceous groups, advantageously to aryls or to alkyls;
- X' is chosen from chloride, bromide, iodide or thiocyanate radicals or sulphonate radicals, advantageously perfluorinated on the carbon carrying the sulphonate functional group;
- X is chosen from the values of X' and from radicals of formula Y-Z;
- Y is chosen from the group of chalcogens, advantageously light chalcogens, that is to say, oxygen and sulphur;
- Z is chosen from the group consisting of trisubstituted tin, monosubstituted zinc and residues of oxygen-comprising acids, the OH functional group not being included.

**2.** Use according to Claim 1, **characterized in that** X has the same value as X'.

**3.** Use according to Claims 1 and 2, **characterized in that** at least one of the X or X' groups is chosen from halides and thiocyanates.

**4.** Use according to Claim 1, **characterized in that** Z has the same value as:

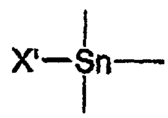

5. Composition, **characterized in that** it comprises at least one carbamate capable of being obtained by the reaction of a hydroxylated compound with an advantageously aliphatic isocyanate and at least one compound of following general formula (I):

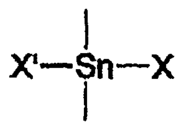 (I)

where:

- The "free" bonds of the tin are connected to hydrocarbonaceous groups, advantageously to aryls or to alkyls;
- X' is chosen from chloride, bromide, iodide or thiocyanate radicals or sulphonate radicals, advantageously perfluorinated on the carbon carrying the sulphonate functional group;
- X is chosen from the values of X' and from radicals of formula Y-Z;
- Y is chosen from the group of chalcogens, advantageously light chalcogens, that is to say, oxygen and sulphur;
- Z is chosen from the group consisting of trisubstituted tin, monosubstituted zinc and residues of oxygen-comprising acids, the OH functional group not being included.

6. Composition according to Claim 5, **characterized in that** it additionally comprises an alcohol or polyalcohol which is advantageously primary.

7. Composition according to Claims 5 and 6,
**characterized in that** said hydroxylated compound is chosen from alcohols which are volatile at a temperature at least equal to 150°C and from hydroxylated masking agents.

8. Composition according to Claims 5 to 7, **characterized in that** said hydroxylated compound is methanol.

9. Composition according to Claims 5 to 7, **characterized in that** said hydroxylated compound is chosen from masking agents, advantageously from phenols and oximes.

10. Transcarbamation process, **characterized in that** use is made, as transcarbamation catalyst, of the compounds of formula (I):

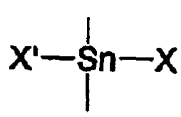 (I)

where:

- The "free" bonds of the tin are connected to hydrocarbonaceous groups, advantageously to aryls or to alkyls;
- X' is chosen from chloride, bromide, iodide or thiocyanate radicals or sulphonate radicals, advantageously perfluorinated on the carbon carrying the sulphonate functional group;
- X is chosen from the values of X' and from radicals of formula Y-Z;
- Y is chosen from the group of chalcogens, advantageously light chalcogens, that is to say, oxygen and sulphur;
- Z is chosen from the group consisting of trisubstituted tin, monosubstituted zinc and residues of oxygen-comprising acids, the OH functional group not being included.

11. Process according to Claim 10, **characterized in that** the transcarbamation reaction is carried out at a temperature of between 100 and 200°C, advantageously between 120 and 180°C.